# EUROPEAN PATENT APPLICATION

(11) **EP 4 269 553 A1**
(43) Date of publication of application: **01.11.2023**
(21) Application number: 21911633.2
(22) Date of filing: 20.12.2021
(51) Int. Cl.: C12M 1/12, C12M 1/00, C12M 3/00

(54) **CELL CULTURE PLATE AND STACKED ARRAY BODY OF CELL CULTURE PLATES**

(30) Priority: 24.12.2020 KR 20200183888
(71) Applicant: Next & Bio Inc., Seoul 08826 (KR)
(72) Inventor: YANG, Ji Hoon, Seoul 04413 (KR); KWAK, Tae Hwan, Seoul 05698 (KR); OH, Sang Taek, Seoul 02714 (KR)
(74) Representative: Kador & Partner Part mbB
(86) International application number: PCT/KR2021/095129
(87) International publication number: WO 2022/139568

(57) **Abstract**

A cell culture plate according to one embodiment of the present invention is a cell culture plate which is formed by side walls and a bottom surface, comprises a space containing a culture medium, has an open top, and is formed to be vertically stacked. The bottom surface includes a well area in which a plurality of wells are formed and a peripheral area formed around the well area, and the cell culture plate according to one embodiment of the present invention includes a culture medium flow hole formed through the bottom surface to allow a culture medium to flow in the peripheral area; a magnetic bead which is provided to open/close the culture medium flow hole by moving up and down due to a magnetic force, and formed of a material responding to magnetic force; and a bead holding space which forms a space allowing the movement of the magnetic bead and prevents the magnetic bead from being separated.

## Description

### [Technical Field]

The present invention relates to a cell culture plate, and more particularly, to a cell culture plate, which is able to be stacked to enable cell culture by an interaction between different types of cells, and a stacked assembly thereof.

### [Background Art]

Technology for culturing cells and tissue is becoming more and more important for basic or applied biological research. Cell culture generally means isolating a piece of tissue from a multicellular organism and culturing and proliferating the piece of tissue in a container.

Direct interactions of specific cells with other types of cells have a great effect on cell growth, migration, and differentiation. For example, the growth rate of cancer cells increases when coexisting with other tissue cells.

A cell culture insert that uses a permeable membrane as a support is mainly used as interactions with other cells are required during cell culture. A membrane is a thin film with fine through-holes through which cells cannot pass, but a culture medium can pass.

A culture insert is inserted into a well of a well plate, first cells are disposed between the lower end of the insert and the bottom surface of a well, and second cells are disposed in the cell culture insert to allow mutual interactions between the first and second cells during culture.

However, in the case of such a known cell culture apparatus, it is difficult to observe cells due to a porous membrane at the lower end of a cell culture insert. In optical observation, since through-holes formed in the membrane cause light scattering, it is difficult to observe the cells cultured in, for example, a cell culture insert.

### [Disclosure]

### [Technical Problems]

The present invention is directed to providing a cell culture plate, and more particularly, to a cell culture plate, which is able to be stacked to enable cell culture by an interaction between different types of cells, and a stacked assembly thereof.

The present invention is also directed to providing a non-stacked, single cell culture plate which enables cell culture and is able to induce interactions between cells by the connection of a culture medium flow path through stacking, and a stacked assembly thereof.

The present invention is also directed to providing a cell culture plate which is able to selectively open a flow path between cell culture plates in which different types of cells are cultured, and a stacked assembly thereof.

### [Technical Solutions]

A cell culture plate according to one embodiment of the present invention is a cell culture plate which is formed by side walls and a bottom surface, includes a space containing a culture medium, has an open top, and is formed to be vertically stacked. The bottom surface includes a well area in which a plurality of wells are formed and a peripheral area formed around the well area, and the cell culture plate according to one embodiment of the present invention includes a culture medium flow hole formed through the bottom surface to allow a culture medium to flow in the peripheral area; a magnetic bead which is provided to open/close the culture medium flow hole by moving up and down due to a magnetic force, and formed of a material responding to magnetic force; and a bead holding space which forms a space allowing the upward and downward movements of the magnetic bead and prevents the magnetic bead from being separated.

According to one embodiment of the present invention, the cell culture plate includes an auxiliary plate having a bottom area bonded with the peripheral area and an opening corresponding to the well area, and in the bottom area, a net-shaped area which covers the culture medium flow hole to form the bead holding space, allows a culture medium to flow, and prevents the magnetic bead from being separated is formed.

According to one embodiment of the present invention, in the peripheral area, an annular bead holding space which surrounds the culture medium flow hole, extends toward an upper opening of the cell culture plate, in which the magnetic bead is located to move up and down, and allows a culture medium to flow in a lateral direction is formed.

According to one embodiment of the present invention, the annular bead holding space is formed by a plurality of pillars arranged to surround the culture medium flow hole in the peripheral area.

According to one embodiment of the present invention, a magnet cover including a magnet member for applying a magnetic force to the magnetic bead is provided at an upper end of the cell culture plate.

According to one embodiment of the present invention, a coupling part for forming liquid tightness by coupling an upper end of the side wall of another cell culture plate stacked above is provided at an edge of the lower end of the bottom surface.

The stacked cell culture plate assembly according to an embodiment of the present invention is a stacked cell culture plate assembly formed by stacking a plurality of cell culture plates in a vertical direction, and includes a magnet cover having a magnet member for applying a magnetic force to the magnetic bead at an upper end of the stacked cell culture plate assembly.

According to one embodiment of the present invention, the stacked cell culture plate assembly may further include an insert stacked with the cell culture plate and formed of a porous membrane on a bottom surface.

According to one embodiment of the present invention, a storage container for accommodating the stacked cell culture plate assembly may be further included.

According to one embodiment of the present invention, a hanging net member in which a plurality of the cell culture plates are accommodated in a stacked form, and a through hole is formed in at least a side of sides and a lower end thereof to allow the access of a manipulation tool or an operator's hand and which has an annular protrusion that protrudes in an annular shape at an upper end thereof and hangs on top of the storage container may be further included.

According to one embodiment of the present invention, a circulator for circulating a culture medium may be further included in the storage container.

### [Advantageous Effects]

The present invention can be applied in various fields such as various cell culture systems, that is, all types of tissue and cells capable of 2D/3D cell culture, and recent organoids. For example, the present invention can be applied to all of embryonic stem cells (ES cells), induced pluripotent stem cells (iPSCs), organoids, and disease mimics based on the cells, and 2D/3D cultured cells capable of mimicking the human body.

The present invention can provide culture conditions for 3D culture similar to the living body, and can sequentially and selectively control the circulation of a culture medium.

In addition, the present invention can be applied to not only cell culture but also to a device that sequentially, selectively and quantitatively controls the transport of various types of liquids in various types of laboratories, factories for producing chemical products, or circulators including a waste water disposal facility.

The stacked cell culture plate assembly according to one embodiment of the present invention enables communication and exchange of culture media between stacked cell culture plates.

The stacked cell culture plate assembly according to one embodiment of the present invention can be a platform capable of evaluating the effectiveness of a novel drug using disease mimics, particularly, using two types of organs and cells, in which information on diseases, particularly, phenotype and microenvironment information is reproduced, through simulation of the structures and functions of living (human) organs. That is, the present invention is integrated platform technology including all of 2D/3D culture conditions optimized according to the form of possessing the ability of each organ.

According to the present invention, for example, in toxicity tests for drugs and chemicals, which must be delivered through blood vessels, a 2D culture insert that embodies blood vessels is required, and a process in which drugs passing through the insert are delivered to nerve cells or brain organoids can be created.

In addition, according to the present invention, it is possible to reproduce the process of reaching the lower respiratory tract (lung) through the upper respiratory tract (nasal mucosa) like the COVID 19 virus.

The present invention can be used as a product using a 2D/3D biomimetic tissue chip for multiple organs (intestines, liver, kidney, etc.), and such technology simulates the absorption, distribution, metabolism and excretion (ADME) of drugs, occurring in human organs, using a multi-organ platform based on multi-organ scaling design, such as the intestines, liver, kidneys, etc. In addition, since real microenvironments in the human body can be simultaneously simulated, the present invention can be used as a technology to analyze drug efficacy and stability for the development of a novel drug, which can be monitored in real time.

According to the present invention, it is possible to provide an effect that replaces animal experiments, and it is possible to establish a toxicity and metabolism evaluation system, which was conducted in animal experiments, and standardize measurements, which was impossible in basic animal experiments. In addition, using 2D/3D biomimetics and disease mimetics, it is possible to present standard protocols for validation and stability evaluation in drug toxicity and metabolism evaluation.

In addition, according to an embodiment of the present invention, since a plurality of cell culture plates can be combined into multiple layers, mass production and culture become possible, and in the case of a well-in-well plate, the standard type or mass culture of subjects (biomimetics or disease mimetics) is enabled.

According to the present invention, different types of cells are allowed to be cultured while interacting with each other. Particularly, cells of one type are allowed to be placed under a mutual communication environment in a state of being individually cultured until the time when an interaction is required, and since the opening/closing of the culture medium flow hole can be adjusted, it is possible to culture cells while appropriately controlling the timing and time of mutual communication.

In addition, since the space in which cells are cultured is formed to be optically observed, various approaches and observations are easy in a culture process in which cells cultured under interaction are individually observed while separating cell culture plates and then the plates are restacked.

### [Description of Drawings]

FIG. 1 is a schematic cross-sectional view of a cell culture plate according to a first embodiment of the present invention.
FIG. 2 is a schematic plan view of a state in which an auxiliary plate is separated from the cell culture plate according to the first embodiment of the present invention.
FIG. 3 is a schematic plan view of an auxiliary plate of the cell culture plate according to the first embodiment of the present invention.
FIG. 4 is a schematic cross-sectional view of a cell culture plate according to a second embodiment of the present invention.
FIG. 5 is a schematic plan view of the cell culture plate according to the second embodiment of the present invention.
FIG. 6 is a view illustrating a stacked cell culture plate assembly according to an embodiment of the present invention.
FIG. 7 is a view illustrating a modified example of the stacked cell culture plate assembly according to the embodiment of the present invention.
FIG. 8 is a view illustrating another modified example of the stacked cell culture plate assembly according to the embodiment of the present invention.

### [Modes of the Invention]

The present invention may have various modifications and various examples, and thus specific examples are illustrated in the drawings and described in detail in the detailed description. However, it should be understood that the present invention is not limited to specific embodiments, and includes all modifications, equivalents or alternatives within the spirit and technical scope of the present invention. In addition, in the drawings, the size, width, length, thickness and the like of components may be exaggerated or arbitrarily expressed for description.

Terms "first" and "second" may be used to describe various components, but the components should not be limited by these terms. These terms are only used for the purpose of distinguishing one component from another.

The terms used herein are used only to describe specific embodiments, not to limit the present invention. Singular expressions include plural expressions unless the context clearly indicates otherwise.

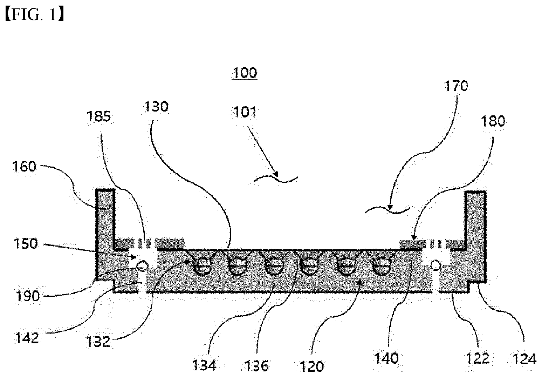

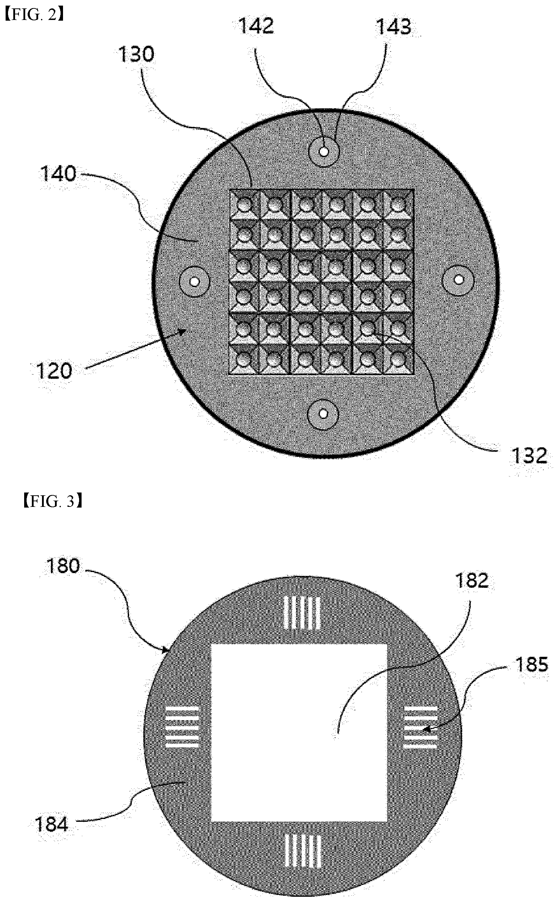

FIG. 1 is a schematic cross-sectional view of a cell culture plate according to a first embodiment of the present invention, FIG. 2 is a schematic plan view of a state in which an auxiliary plate is separated from cell culture plate according to the first embodiment of the present invention, and FIG. 3 is a schematic plan view of an auxiliary plate of the cell culture plate according to the first embodiment of the present invention.

Although FIGS. 1 to 3 are expressed separately to easily distinguish components, the cell culture plate is formed transparently. In addition, the cell culture plate is formed of a biocompatible material. Unless particularly limited, the cell culture plate can be formed of a transparent biocompatible plastic such as PDMS, PMMA, PET, or PC, and manufactured by injection molding.

Referring to FIGS. 1 to 3, the cell culture plate 100 according to the first embodiment of the present invention includes a space 170 formed by a bottom surface 120 and a side wall 160 surrounding the bottom surface 120. The space 170 forms a space filled with a culture medium. The upper portion of the cell culture plate 100 is open to form an upper opening 101.

The bottom surface 120 includes a well area 130 in which a plurality of wells is formed, and a peripheral area 140 formed around the well area.

A plurality of wells 132 are formed in the well area 130. Each well 132 may be formed with a concave part 134 and an inlet inclined surface 136 inclined so as to be tapered toward the upper entrance of the concave part 134.

The horizontal area of the upper portion of the well 132 may decrease as it descends in a vertical direction. The well 132 may be formed in a pyramid shape with a square-shaped top, or a shape whose horizontal area decreases as it descends in the vertical direction, like a funnel shape.

Since there are a plurality of wells 132 with the same size and shape, a cell culture plate is formed to culture cell aggregates, such as spheroids or organoids, under uniform conditions.

The well 132 includes the concave part 134 as a space for culturing 3D spheroids or organoids in the lower end. The concave part 134 may have a U shape, a V shape, a cup shape, and a hemispherical shape.

The concave part 134 may have a volume of 20 to 50 µL. The concave part 134 is a substantial cell culture space, and when the volume is less than 20 µL, a problem in that cells escape due to insufficient cell culture space may occur, and when the volume is more than 50 µL, it is difficult to stably culture cells.

The well 132 is able to culture 100 cells per well, and when 100 cells/well or less are seeded, the cells can be stably controlled.

The upper diameter of the well 132 may be 3.0 to 4.5 mm, 3.5 to 4.3 mm, or 4 mm. In addition, the diameter of the concave part 134 may be 0.45 to 1.5 mm, 0.5 to 1.0 mm, or an average of 0.5 mm.

In addition, the length ratio of the diameter of the well 132 and the diameter of the concave part 134 may be 1:0.1 to 0.5, and preferably, the length ratio of the diameter of the well 132 and the diameter of the concave part 134 may be 1: 0.12.

The inlet inclined surface 136, surrounding the concave part 134, may have the angle of inclination of 40 to 50°, 42 to 48°, 43 to 47°, or an average angle of inclination of 45°, based on the vertical direction.

According to the present invention, the upper end of the inlet inclined surface 136 of each well is formed in contact with each other. Since the inlet inclined surface 136 has an inclined surface, and the upper ends of adjacent wells 132 are in contact with each other, cells are introduced into the well 132 without adhering to an interwell surface during cell seeding. Therefore, this enables uniform seeding of cells.

However, the cell culture plate according to the present invention is not limited by the shape of the well 132 formed in the well area 130, and a plurality of wells formed in the well area may be formed different structures.

According to one embodiment of the present invention, in the peripheral area 140 of the bottom surface 120 of the cell culture plate 100, a culture medium flow hole 142 is formed through the bottom surface 120. The culture medium flow hole 142 becomes a path for discharging a culture medium in the space 170 of the cell culture plate 100 downward. The culture medium flow hole 142 may be formed to have a linear flow path.

The culture medium flow hole 142 is provided with a bead holding space 150 for supporting the upward and downward movements of a magnetic bead 190 and preventing its outward movement.

The magnetic bead 190 is provided to control the opening/closing of the culture medium flow hole 142. When the magnetic bead 190 is located in a lowered position blocking an inlet of the culture medium flow hole 142, the culture medium flow hole 142 is closed, and when the magnetic bead 190 is located in an elevated position spaced apart from the inlet of the culture medium flow hole 142, the culture medium flow hole 142 is open, allowing the culture medium to flow downward and allowing communication between the culture media in upper and lower cell culture plates 100 when the cell culture plates 100 are stacked vertically.

The magnetic bead 190 is formed to be movable in the direction of a magnetic force by an applied magnetic force, and may be formed of a metal material or magnetic material responding to magnetic force. The surface of the magnetic bead 190 may be coated with a biocompatible material.

According to the first embodiment of the present invention, the cell culture plate 100 includes an auxiliary plate 180, and the bead holding space 150 is formed by the auxiliary plate 180.

As shown in FIG. 3, the auxiliary plate 180 includes an opening 182 corresponding to the well area 130 and a bottom area 184 corresponding to the peripheral area 140, and a net-shaped area 185 is formed at a location corresponding to the culture medium flow hole 142. The net-shaped area 185 may be formed in various shapes such as holes and slits, which allow fluid movement and prevent the movement of the magnetic bead 190.

According to the first embodiment of the present invention, the bead holding space 150 is defined as an area covered by the net-shaped area 185 in which the magnetic bead 190 moves, and the magnetic bead 190 is maintained in the bead holding space 150, and opens/closes the culture medium flow hole 142 by ascending movement and descending movement caused by a load.

When the net-shaped area 185 is formed in contact with the bottom surface 120, an enlarged hole area 143 allowing the movement of the magnetic bead 190 is formed at the top of the culture medium flow hole 142, and a bead holding space 150, surrounded by the enlarged hole area and the net-shaped area 185, is formed. When the net-shaped area 185 is formed in a protruding shape to allow the movement of the magnetic bead 190, a separate enlarged hole area 143 may not be formed.

The auxiliary plate 180 is adhered to the bottom surface 120 of the cell culture plate 100 using an adhesive, thereby forming a cell culture plate 100 as an assembled whole.

According to the first embodiment of the present invention, the cell culture plate 100 is formed such that the upper and lower cell culture plates are liquid-tightly bonded when arranged in a stacked manner. To this end, an edge of the lower end 122 of the bottom surface 120 of the cell culture plate 100 is formed with a single-step coupling part 124 into which the upper end of the side wall 160 of the cell culture plate is fitted. A liquid-tight effect may be improved by coating the coupling part 124. As a result, it is possible to prevent or minimize the leakage of the culture medium between the coupling parts between cell culture plates when the cell culture plates 100 are stacked. Preferably, complete liquid tightness can be achieved.

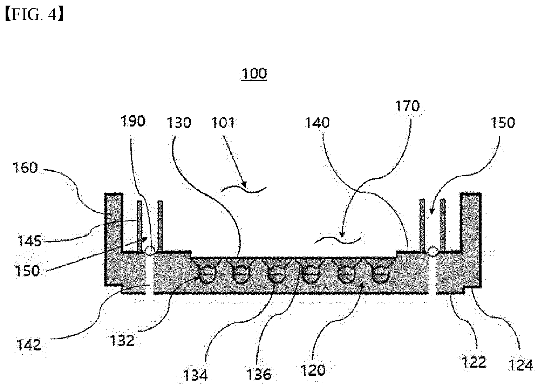

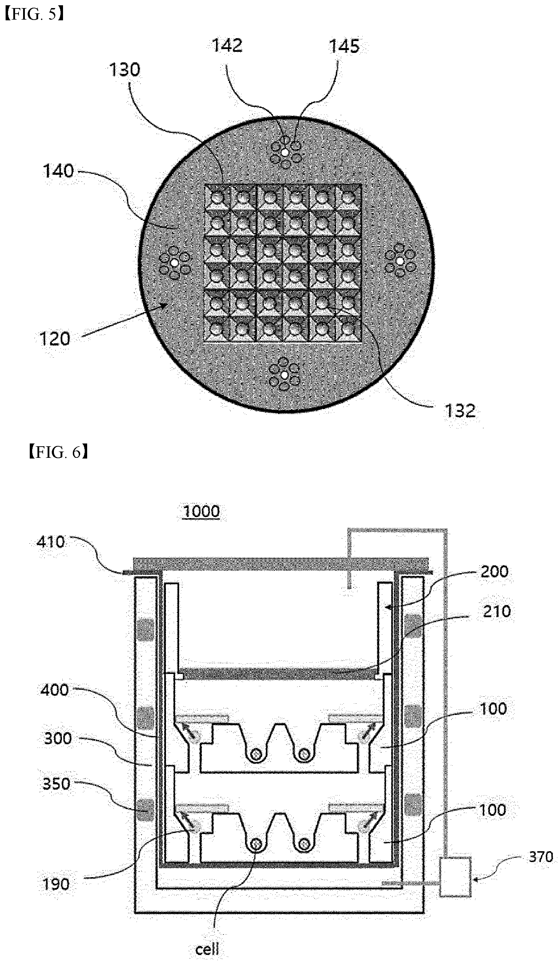

FIG. 4 is a cross-sectional view of a cell culture plate according to a second embodiment of the present invention, and FIG. 5 is a plan view of the cell culture plate according to the second embodiment of the present invention. In description of the second embodiment of the present invention, descriptions of partis identical and similar to those of the first embodiment of the present invention are omitted.

Referring to FIGS. 4 and 5, in a cell culture plate 100 according to the second embodiment of the present invention, a bead holding space 150 is formed by arranging a plurality of pillars 145. The pillars 145 are installed to extend toward an upper opening 101 of the cell culture plate 100 from the periphery of a culture medium flow hole 142, and disposed in an annular shape surrounding the culture medium flow hole 142. The arrangement of pillars 145 forms a bead holding space 150 extending upward, a culture medium is allowed to flow from a space 170 to the culture medium flow hole 142 via the bead holding space 150 through a gap between the pillars 145.

According to the second embodiment of the present invention, in the bead holding space 150, since a magnetic bead 190 moves up and down in the space surrounded by the pillars 145, and the pillars 145 are extended to a location adjacent to the upper opening 101, when the cell culture plate 100 is covered with a cover (not shown), or arranged in a stacked form, a lower end 122 of the bottom surface 120 of an upper cell culture plate blocks the top of the bead holding space 150, thereby preventing the separation of the magnetic bead 190.

According to a modified example of the present invention, the bead holding space 150 may be formed using an annular member having a hollow and a cut side instead of the pillars 145. The annular bead holding space 150 is positioned inside so that the magnetic bead can move up and down and the culture medium is allowed to flow in a lateral direction.

FIG. 6 is a view illustrating a stacked cell culture plate assembly according to an embodiment of the present invention. While the cell culture plate shown in FIG. 6 is illustrated as the cell culture plate according to the first embodiment of the present invention, the cell culture plate according to the second embodiment is also applied in the same manner.

Referring to FIG. 6, a stacked cell culture plate assembly 1000 may include a storage container 300 for accommodating the stacked cell culture plates.

A plurality of cell culture plates 100 are stacked and accommodated in the storage container 300. The storage container 300 may contain a culture medium discharged from the disposed cell storage container at the bottom when a culture medium flow hole 142 of each cell culture plate is open, and even when the culture medium flow hole 142 is open, the culture medium in a space of the upper cell culture plate may be maintained.

According to one embodiment of the present invention, for smooth handling of the stacked cell culture plate assembly, a hanging net member 400 holding the stacked cell culture plate assembly may be further included.

The hanging net member 400 has an annular protrusion 410 at an upper side of a main body with an open top, and the sides and lower end forming the main body are perforated by ribs. The ribs are loosely formed such that the access of an operator's hand or a manipulation tool is allowed through a space or through hole between the ribs. Accordingly, the accommodation and withdrawal of the stacked cell culture plate assembly may be facilitated, and it is easy to accommodate or withdraw the stacked cell culture plate assembly in or from the storage container. The annular protrusion 410 hangs on top of the storage container. The hanging net member may be prepared of a biocompatible plastic such as PDMS, PMMA, PET, or PC.

A magnet cover 350 is disposed on top of the storage container 300. The magnet cover 350 includes a magnet member 355 capable of applying a magnetic force to a magnetic bead 190 positioned above the culture medium flow hole 142. The magnet member 355 may be an electromagnet or a permanent magnet.

As shown in FIG. 6, when a magnetic force is applied by the magnet member 355 of the magnet cover 350 while the stacked cell culture plate assembly 1000 is accommodated in the storage container 300, the magnetic bead 190 moves upward due to the magnetic force, and the culture medium flow hole 142 is open. Accordingly, while culture media communicate between a lower cell culture plate, an intermediate cell culture plate, and an upper cell culture plate, an interaction between different cells is allowed. That is, in an environment allowing an interaction between cells, cell culture is performed. The stacked cell culture plate assembly 1000 may be used, for example, in a cell culture model mimicking human body systems, such as a vertical cell culture model of a digestive system, or a pharmacological effect experiment, etc.

The stacked cell culture plate assembly 1000 may properly control the culture medium flow between the culture plates. That is, when the magnetic force is removed through the separation of the magnet cover 350 or the power OFF of an electromagnet, the magnetic bead 190 falls by its load to close the culture medium flow hole 142. The flow of the culture media between cells and materials included in the culture media may be intermittently controlled.

According to an embodiment of the present invention, the stacked cell culture plate assembly 1000 may further include a circulator 370 for circulating culture media. The circulator 370 may be advantageously applied to cell culture requiring the circulation of culture media, and the exchange of culture media may be performed by connecting a culture medium container to the circulator 370.

FIG. 6 illustrates that the stacked cell culture plate assembly 1000 is used after being formed by stacking a plurality of cell culture plates 100 according to one embodiment of the present invention.

However, the cell culture plate 100 according to the present invention is not limited by being used in a stacked form. For example, a single cell culture plate 100 may be used with a magnet cover 350. When a magnetic force is applied by the magnet cover 350, the culture medium is discharged while the culture medium flow hole 142 is open. That is, in the case of a conventional cell culture plate, although injection and discharge were performed by pipetting in the exchange of a culture medium, in the cell culture plate 100 according to an embodiment of the present invention, the discharge of a culture medium may be performed without pipetting. Accordingly, in the discharge of a culture medium, the problem in that cell aggregates, such as spheroids or organoids, in culture are sucked up or translocated by pipetting may be fundamentally prevented, and only injection of a culture medium may be performed by pipetting. In addition, a cell culture plate containing only a culture medium may be stacked on top, the injection and discharge of the culture medium are performed simultaneously, and the exchange of the culture medium may be performed.

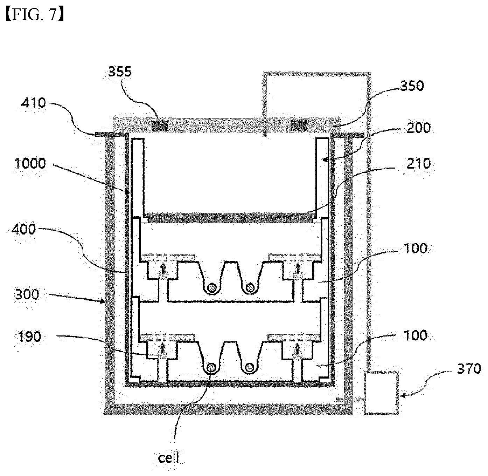

FIG. 7 is a view illustrating a modified example of the stacked cell culture plate assembly according to the embodiment of the present invention.

As shown in FIG. 7, in the stacked cell culture plate assembly, a cell culture plate according to an embodiment of the present invention may be stacked with an insert 200 in the form capable of being matched with the cell culture plate. The insert 200 may have a porous membrane 210 included in the lower end of the main body, and for example, when it is necessary to provide a culture medium that has passed through a blood cell layer into the cell culture plate, the blood cell layer may be accommodated on the surface of the porous membrane 210, and the culture medium may flow into a lower cell culture plate via the blood cell layer.

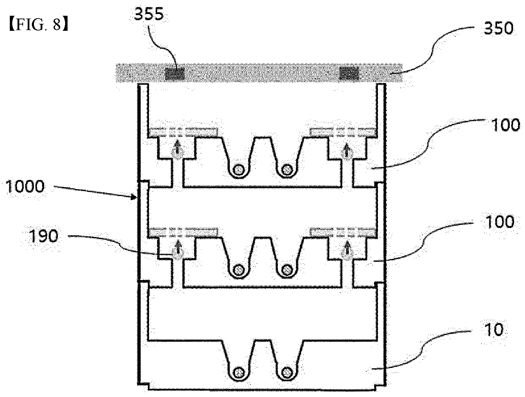

FIG. 8 is a view illustrating another modified example of the stacked cell culture plate assembly according to the embodiment of the present invention.

The cell culture plate shown in FIG. 8 is in a stacked assembly, and unlike the embodiments shown in FIGS. 6 and 7, a cell culture plate 10 disposed at the bottom may be formed in a structure without a culture medium flow hole, or the structure of a cell culture plate 100 in which a culture medium flow hole 142 is blocked using a nonmagnetic member.

Therefore, cell culture plates can be stacked without using a storage container, and interactions between cells cultured in different cell culture plates are possible by allowing the flow of a culture medium between the stacked cell culture plates by opening a culture medium flow hole.

The scope of protection described in the claims of the present invention is not limited to the description and expression of the embodiments explicitly described above. In addition, the scope of protection of the present invention may not be limited due to obvious changes or substitutions in the technical field to which the present invention belongs.

### <Description of reference numerals>

100: Cell culture plate
101: Upper opening
120: Bottom surface
124: Coupling part
130: Well area
132: Well
134: Concave part
136: Inlet inclined surface
140: Peripheral area
142: Culture medium flow hole
145: Pillar
150: Bead holding space
160: Side wall
170: Space
180: Auxiliary plate
182: Opening
184: Bottom area
185: Net-shaped area
200: Insert
210: Porous membrane
300: Storage container
350: Magnet cover
355: Magnet member
400: Hanging net member
410: Annular protrusion
1000: Stacked cell culture plate assembly

## Claims

1. A cell culture plate which is formed by side walls and a bottom surface, comprises a space containing a culture medium, has an open top, and is formed to be vertically stacked,
wherein the bottom surface has a well area in which a plurality of wells are formed and a peripheral area formed around the well area, and
the cell culture plate has a culture medium flow hole formed through the bottom surface to allow a culture medium to flow in the peripheral area;
a magnetic bead which is provided to open/close the culture medium flow hole by movement due to a magnetic force, and formed of a material responding to magnetic force; and
a bead holding space which forms a space allowing the movement of the magnetic bead and prevents the magnetic bead from being separated.

2. The cell culture plate of claim 1, further comprising:
an auxiliary plate having a bottom area bonded with the peripheral area and an opening corresponding to the well area,
wherein, in the bottom area, a net-shaped area which covers the culture medium flow hole to form the bead holding space, allows a culture medium to flow, and prevents the magnetic bead from being separated is formed.

3. The cell culture plate of claim 1, wherein, in the peripheral area, an annular bead holding space which surrounds the culture medium flow hole, extends toward an upper opening of the cell culture plate, in which the magnetic bead is located to move up and down, and allows a culture medium to flow in a lateral direction is formed.

4. The cell culture plate of claim 3, wherein the annular bead holding space is formed by a plurality of pillars arranged to surround the culture medium flow hole in the peripheral area.

5. The cell culture plate of claim 1, wherein a magnet cover including a magnet member for applying a magnetic force to the magnetic bead is provided at an upper end of the cell culture plate.

6. The cell culture plate of claim 1, wherein a coupling part for forming liquid tightness by coupling an upper end of the side wall of another cell culture plate stacked above is provided at an edge of the lower end of the bottom surface.

7. A stacked cell culture plate assembly formed by stacking a plurality of cell culture plates according to any one of claims 1 to 4, and 6 in a vertical direction,
wherein a magnet cover having a magnet member for applying a magnetic force to the magnetic bead is provided at an upper end of the stacked cell culture plate assembly.

8. The stacked cell culture plate assembly of claim 7, further comprising:
an insert that is stacked with the cell culture plate, and formed of a porous membrane on a bottom surface.

9. The stacked cell culture plate assembly of claim 7, further comprising:
a storage container that accommodates the stacked cell culture plate assembly.

10. The stacked cell culture plate assembly of claim 9, further comprising:
a hanging net member in which a plurality of the cell culture plates are accommodated in a stacked form, and a through hole is formed in at least a side of sides and a lower end thereof to allow the access of a manipulation tool or an operator's hand, and which has an annular protrusion that protrudes in an annular shape at an upper end thereof and hangs on top of the he storage container.

11. The stacked cell culture plate assembly of claim 7, wherein the storage container comprises a circulator for circulating a culture medium.
